(19) Europäisches Patentamt
European Patent Office
Office européen des brevets

(11) Publication number: **0 505 220 A1**

# (12) EUROPEAN PATENT APPLICATION

(21) Application number: **92302491.3**

(22) Date of filing: **23.03.92**

(51) Int. Cl.[5]: **C07D 251/70,** A61K 31/53

(30) Priority: **22.03.91 GB 9106170**

(43) Date of publication of application:
**23.09.92 Bulletin 92/39**

(84) Designated Contracting States:
**AT BE CH DE DK ES FR GB GR IT LI LU NL PT SE**

(71) Applicant: **JARMAN, Michael**
**The Institute of Cancer Research, Royal Cancer Hospital, 15 Cotswold Road Belmont Sutton, Surrey SM2 5NG (GB)**

(71) Applicant: **Wilman, Derry Edward Vincent**
**The Institute of Cancer Research, Royal Cancer Hospital, 15 Cotswold Road Belmont Sutton, Surrey SM2 5NG (GB)**

(71) Applicant: **Judson, Ian Robert**
**The Institute of Cancer Research, Royal Cancer Hospital, 15 Cotswold Road Belmont Sutton, Surrey SM2 5NG (GB)**

(72) Inventor: **JARMAN, Michael**
**The Institute of Cancer Research, Royal Cancer Hospital, 15 Cotswold Road Belmont Sutton, Surrey SM2 5NG (GB)**
Inventor: **Wilman, Derry Edward Vincent**
**The Institute of Cancer Research, Royal Cancer Hospital, 15 Cotswold Road Belmont Sutton, Surrey SM2 5NG (GB)**
Inventor: **Judson, Ian Robert**
**The Institute of Cancer Research, Royal Cancer Hospital, 15 Cotswold Road Belmont Sutton, Surrey SM2 5NG (GB)**

(74) Representative: **Goldin, Douglas Michael et al**
**J.A. KEMP & CO. 14, South Square Gray's Inn London WC1R 5LX (GB)**

## (54) New compounds for use in the treatment of cancer.

(57) Compounds of the formula:

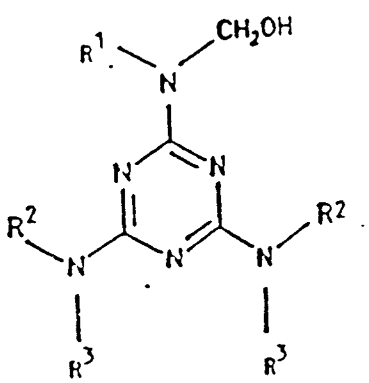

I

wherein $R^3$ is hydrogen, alkyl or hydroxymethyl, and $R^1$ and $R^2$ are hydrogen, alkyl or an electron withdrawing organic group containing at least two carbon atoms, at least one of $R^1$ and $R^2$ being an electron withdrawing group, and with the proviso that: when $R^3$ is hydroxymethyl, $R^1$ and $R^2$ are electron withdrawing organic groups; are useful in the treatment of cancers, particularly cis-platin resistant ovarian cancer. The new compounds are obtainable by reacting formaldehyde with an intermediate of formula:

EP 0 505 220 A1

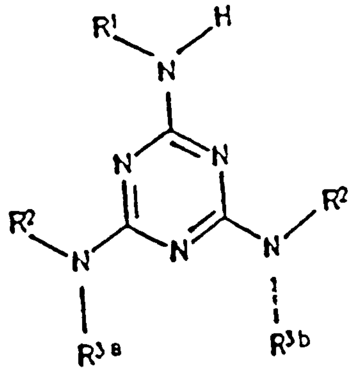

II

wherein $R^{3a}$ and $R^{3b}$ are hydrogen, alkyl or hydroxymethyl, but not both hydroxymethyl, and $R^1$ and $R^2$ are hydrogen, alkyl or an electron withdrawing organic group containing at least two carbon atoms, and at least one of $R^1$ and $R^2$ is an electron-withdrawing organic group and where necessary, converting any N-hydroxy-methoxymethyl substituents to N-hydroxymethyl by treatment with an aqueous medium.

This invention relates to novel 2,4,6-triamino-1,3,5-triazines, compositions containing them, processes for making them and their use in the treatment of carcinomas, particularly ovarian carcinomas.

Trimelamol (1) [2,4,6-tris{(hydroxymethyl)(methyl) amino}-1,3,5-triazine] is clinically active, particularly against ovarian carcinomas, but its clinical development has been halted due to difficulties with formulation due to instability with respect to the formation of dimers during formulation.

We have studied new analogues of the trimelamol-type with a view to identifying compounds having a similar level of activity against carcinomas, particularly ovarian carcinomas, but which are more stable and hence more amenable to formulation.

It has been established that the half-life of trimelamol in humans is short and this may limit its clinical efficacy (reference I.R. Judson, et al. Cancer Res. 49, 5475-5479, 1989). We believe that this is, in part, due to the chemical instability of the N-hydroxymethyl functions resulting in the release of formaldehyde. We have investigated stabilizing these functions using electron-withdrawing organic groups (defined in the present context as electron-withdrawing relative to methyl), with a view to lengthening the half-life and also improving amenability to formulation.

Accordingly this invention provides novel 2,4,6-triamino-1,3,5-triazines having the following general formula:

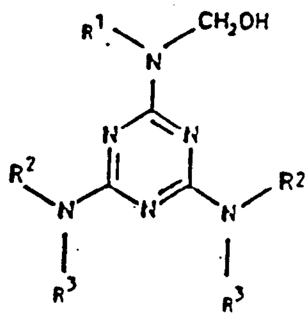

I

wherein $R^3$ is hydrogen, alkyl or hydroxymethyl, and $R^1$ and $R^2$ are hydrogen, alkyl or an electron-withdrawing organic group containing at least two carbon atoms, at least one of $R^1$ and $R^2$ being an electron withdrawing group, and with the proviso that:
when $R^3$ is hydroxymethyl, $R^1$ and $R^2$ are electron-withdrawing organic groups.

Preferred electron-withdrawing organic groups are $-CH_2CF_3$ and $-CH_2C{\equiv}CH$.

The compounds of the present invention are prepared via novel intermediate compounds of the general formula:

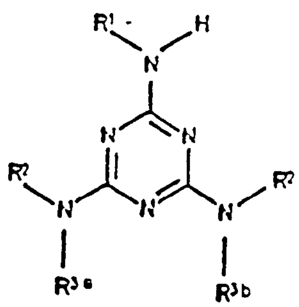

II

wherein $R^3a$ and $R^{3b}$ are hydrogen, alkyl or hydroxymethyl, but not both hydroxymethyl, and
$R^1$ and $R^2$ are hydrogen, alkyl or an electron-withdrawing organic group which is an electron-withdrawing organic group containing at least two carbon atoms, and at least one of $R^1$ and $R^2$ being an electron-withdrawing organic group.

The intermediates are prepared by reacting a cyanuric halide of general formula:

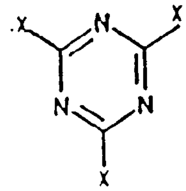

III

wherein X is fluoro or chloro with an amine of the formula $R^1$-$NH_2$ or $R^2$-$NH_2$, wherein $R^1$ or $R^2$ are as defined in formula (I), optionally in the presence of caesium fluoride.

In the absence of caesium fluoride, less than three of the substituents on the 1,3,5-triazine ring may be displaced, which allows for the preparation of asymmetrical compounds.

Treatment of the intermediates II with aqueous formaldehyde, optionally in the presence of potassium carbonate, gives the compounds of formula (I). In the specific case where the intermediate II has the composition $R^1$=$R^2$=$CH_2CF_3$, $R^3$=H and the reaction with aqueous formaldehyde is carried out in the presence of potassium carbonate then the product I of formula $R^1$=$R^2$=$CH_2CF_3$, $R^3$=$CH_2OH$ reacts further to give a material in which one of the 3 $CH_2OH$ groupings is modified by conversion to a $CH_2OCH_2OH$ grouping. This modified product is readily reconverted into the appropriate product I of the invention by treatment with aqueous acetone or other suitable aqueous media, and may be regarded as a pro-drug form of that product in biological test systems.

The compounds of this invention are clinically active and are of use against ovarian carcinomas, particularly against cisplatin-resistant ovarian carcinomas (see Table 3 below).

Also included within the scope of the present invention are pharmaceutical compositions which comprise, as active ingredient, at least one compound of general formula I, in association with a pharmaceutically acceptable carrier or diluent.

The compounds of the invention will normally be administered orally or by injection.

Compositions for parenteral administration will normally be solutions in aqueous saline, which is pyrogen free for human use. Such compositions can be administered intravenously or intraperitoneally.

Compositions for oral administration will mostly be in solid or liquid form, mostly as tablets, capsules, lozenges, etc. Liquid compositions can be solutions or dispersions in aqueous or non-aqueous media. Ideal solutions are of neutral or alkaline pH and of low ionic strength e.g. 5% dextrose.

Suitable daily doses of the compounds of the invention in therapeutic treatment of the human or animal body range from about 100mg to 3g/$m^2$ body-surface.

The following Examples illustrate the preparation of the compounds of the present invention.

EXAMPLE 1

2-Fluoro-4,6-bis[(2,2,2-trifluoroethyl)amino]-1,3,5-triazine(2).

To cyanuric fluoride (1.62g, 12 mmol) was added with stirring at 0°C trifluoroethylamine (3.17g, 24mmol). When the initially vigorous reaction had subsided the mixture was placed under vacuum (rotary evaporator) to remove excess reagents. The resulting white solid (3.41g, 97%) gave colourless crystals from EtOAc:subl. 85°C; m/z 293 ($M^+$,56%), 274 (40%), 273 (52%) and 224 (100%).

EXAMPLE 2

2,4,6-Tris[(2,2,2-Trifluoroethyl)amino]-1,3,5-triazine(3).

To a stirred solution of cyanuric fluoride (2.025g, 15 mmol) in dry DMF (12.5ml) was added with stirring through a septum attached to the top of a reflux condenser 2,2,2-trifluoroethylamine (7.45g, 75 mmol). When the initially vigorous reaction had subsided CsF (5g) was added and the mixture stirred at 40°C for 3 days. The slurry was then poured onto ice (100g) and (3) was recovered as a white solid after washing with ice-cold water and desiccation (5.08g, 91%); $\delta_H$($CDCl_3$) 4.05 (m,6,$CH_2$), 7.42, 7.57 (2 br s, 3, NH); $\delta_F$ -70.3 (s, $CF_3$); m/z (EI)372($M^+$). This product was used directly for the preparation described in Example 5.

EXAMPLE 3

Alternative Method for compound (3), 2,4,6-Tris[(2,2,2,-trifluoroethyl)amino]-1,3,5-triazine.

To a stirred solution of cyanuric fluoride (1.56g, 0.85 ml, 11.53 mmol) in dry toluene (50ml) was added trifluoroethylamine (6.89 g, 69.2 mmol). When the initially vigorous reaction had subsided the mixture was heated under reflux for 20 h. Solid was removed by filtration and the filtrate concentrated. Crystallisation of the residue from acetic acid (25ml): water (20ml) afforded (3) in two crops (2.24g, 52%) having mp 73-75°C, an NMR spectrum identical with the product from Example 2 and analytical data given in Table 1.

EXAMPLE 4

2-(Hydroxymethyl)(2,2,2-trifluoroethyl)amino-4,6-bis[(2,2,2-trifluoroethyl)amino]-1,3,5-triazine (4).

A mixture of (3) (744 mg, 2 mmol) and 40% aqueous formaldehyde adjusted buffered to pH 8.9 with M aqueous NaOH (7.5 ml) was stirred for 16 h. Complete dissolution took 5 min but (4) separated subsequently and was recovered as a white solid after washing first with the formaldehyde solution, then with ice-cold water, then desiccation at 5°C (585 mg, 73%). Recrystallization from $CH_2Cl_2$ gave colourless crystals (345 mg) subl. 135°C; $\delta_H$($Me_2SO$-$d_6$) 4.06 (app t, 4, $NHC\underline{H}_2CF_3$, slight sharpening on $D_2O$ shake to app q), 4.41 (q, $\underline{J}$=9.5 Hz, 2, $CH_2NC\underline{H}_2CF_3$), 5.07 (d,$\underline{J}$=6.3 Hz, 2, $C\underline{H}_2OH$, s on $D_2O$ shake), 5.74 (t, 1, OH), 7.69 (br s, 2, NH); $\delta_F$ - 68.4 (app S, 3, $CH_2NCH_2CF_3$), -70.32 (app s, 6, $NHCH_2CF_3$); $\underline{m/z}$ (FAB, matrix PEG) 403 ($[M+H]^+$,35%), 385 ($[M+H-H_2O]^+$,100%),373 ($[M+H-CH_2O]^+$,68%).

EXAMPLE 5

2,4,6-Tris[(Hydroxymethyl)(2,2,2-trifluoroethyl)amino]-1,3,5-triazine (5).

To a stirred solution of $K_2CO_3$ (829 mg, 6 mmol) in 40% aqueous formaldehyde adjusted to pH 8.9 (20ml) was added (3) (2.231g, 6mmol). The mixture was stirred for 3 days and the resulting white solid was recovered by filtration, washed with aqueous formaldehyde then with ice cold water then desiccated at 5°C to give 2-[([hydroxymethoxy]methyl)(2,2,2-trifluoroethyl)amino]-4,6-bis(hydroxymethyl)(2,2,2-trifluoroethyl)-amino-1,3,5-tria zine(6). (1.81 g, 61%); $\delta_H$ ($Me_2SO$-$d_6$) 4.45 (brq, 6, $C\underline{H}_2CF_3$), 4.65 (d, 2,$\underline{J}$ = 7.6 Hz, $OC\underline{H}_2OH$), 5.06 (br, t, 4, $NC\underline{H}_2OH$), 5.21, 5.25 (2s, total 2, $NC\underline{H}_2OCH_2OOH$), 5.90 (2t, 2, $NCH_2O\underline{H}$), 6.39, 6.59 (2t, total 1, J = 8.5 Hz, $OCH_2O\underline{H}$; $\delta_F$-67.9, -68.1 (2t, $HOCH_2OCH_2NCH_2CF_3$), -68.5 (m, $HOCH_2NCH_2CF_3$). A solution of the intermediate (6) (1.66 g, 3.59 mmol) in acetone (10ml) was treated with water (6.66 ml) then set aside for 1 h at room temperature. Acetone was removed under vacuum and the resulting white precipitate recovered by filtration, washed with water and dried under vacuum over $CaCl_2$ to give the title compound 5 (1.54 g, 99% from the intermediate); $\delta_H$ ($Me_2SO$-$d_6$) 4.45 (2 overlapping q,6, $C\underline{H}_2CF_3$), 5.06 (2d, 6, $C\underline{H}_2OH$), 5.87 (2t, 3, $O\underline{H}$); $\delta_F$-68.4 (m), $CH_2C\underline{F}_3$).

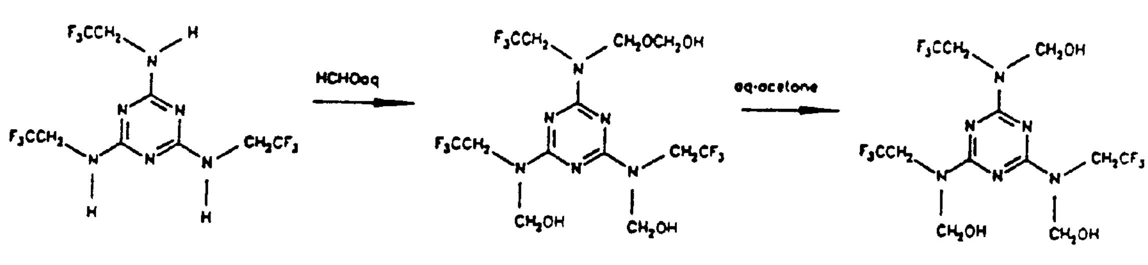


3 6 5

EXAMPLE 6

2-(Hydroxyethyl)(2,2,2-trifluoroethyl)amino-4-methyl-6-(2,2,2-trifluoroethyl)amino-1,3,5-triazine (7).

A mixture of (2) (2.34 g, 8 mmol) and 30% aqueous methylamine (40ml) was stirred for 16 h. The supernatant was decanted from the oil which separated and was then stirred with $H_2O$ (50ml) and the process repeated. After two further additions of $H_2O$ and decantation the oil was triturated with ice-water whereupon crude 2-methylamino-4,6-bis[(2,2,2-trifluoroethyl)amino]-1,3,5-triazine (8) was obtained as a white solid which upon filtration and vaccum desiccation afforded a hygroscopic glass (1.26g, 52%); m/z 304 ($M^+$, 100%) which was used without further purification to prepare (7). A mixture of (8) (304 mg, 1 mmol) and 40% aqueous formaldehyde adjusted to pH 8.9 (5ml) was stirred for 1 h whereupon (7) separated as a white solid (446 mg, 51%) which after 24 h was recovered by washing first with aqueous formaldehyde, then with ice-cold water, then desiccation at 5°C; $\delta_H(Me_2SO-d_6)$ 2.75 (s, 3, $CH_3$), 4.07 (br s, 3, $HNCH_2CF_3$), 4.40 (m, 2, $HOCH_2NCH_2CF_3$), 4.84 (d, 2, $CH_2OH$), 5.74 (m, OH), 7.00 (m, 1, $NHCH_3$), 7.64 (m, 1, $NHCH_2CF_3$); $\delta_F$-69.4 (app s, 3, $CH_2NCH_2CF_3$), -71.2 (app s, 3, $NCH_2CF_3$).

EXAMPLE 7

2,4-Bis(Dimethylamino)-6-(2,2,2-trifluoroethyl)amino-1,3,5-triazine (9).

A solution of 2,4-dichloro-6-(2,2,2-trifluoroethyl)amino-1,3,5-triazine (reference E. Kuhle et al. Chem. Abstr. (1984), 100, 12115h) (3.1 g, 0.013 mol) in aqueous dimethylamine (25% w/v, 200 ml) was heated under reflux, first alone, then when TLC indicated incomplete reaction, with addition of NaOH (1.2g, 0.03 mol). The solid obtained on filtering the cooled reaction mixture was recrystallized from light petroleum (bp 60-80°C) to yield (9) (1.8 g, 528); mp 100°C; $\delta_H(CDCl_3)$ 3.09 (s, 12, $CH_3$), 4.10 (m, 2, $CH_2CF_3$), 4.89 (br t, 1, NH); $\delta_F$-73.2 (t, $J_{F,H}$=9.4Hz, $CF_3$).

EXAMPLE 8

2,4-Bis(Dimethylamino)-6-(hydroxymethyl)(2,2,2,trifluoroethyl)-amino-1,3,5-triazine (10).

A saturated solution of (9) (300 mg) in $H_2O$ (1600 ml) and 0.1 NaOH (10ml) was prepared by overnight stirring at room temperature. Further 0.1 M NaOH (10ml) was added to the filtered solution to bring the pH to 10.5 then 40% aqueous formaldehyde adjusted to pH 8.5, (25ml) was added to give a final pH of 10 and the mixture stirred overnight whereupon (10) separated as a white solid which was recovered by washing with ice-cold water and air drying (50 mg): mp 101-103°C.

EXAMPLE 9

2-Fluoro-4,6-bis(propargylamino)-1,3,5-triazine (11).

To a stirred solution of cyanuric fluoride (810 mg, 6 mmol) in dry DMF (5ml) was added propargylamine (660 mg, 12 mmol). After the initial reaction had subsided further propargylamine (330 mg, 6 mmol) was added. When the further reaction had subsided the resulting white solid was recovered by filtration, washed with DMF, then with water and desiccated. Yield of (11) 333 mg: subl 157°C; m/z (CI) 206 ($[M+H]^+$,100%). A further 743 mg of product was recovered from the filtrate by precipitation with water (20ml) making a total yield for (11) of 1.076 g (87%).

EXAMPLE 10

2,4,6-Tris(propargylamino)-1,3,5-triazine (12).

To a stirred solution of cyanuric fluoride (1.35g, 10 mmol) in toluene (20ml) was added a solution of propargylamine (3.30g, 60 mmol) in toluene (30 ml) during 5 min. The mixture was heated under reflux for 3 h then filtered hot. The filtrate deposited (12) as pale buff crystals (2.225g, 938) : mp 125-127°C; $\upsilon_{max}$ (film from $CH_2Cl_2$) 2116 $cm^{-1}$ (C≡C str); $\delta_H$ $(CDCl_3)$ 2.22 (t, J=2.5 Hz, 3, C≡CH), 4.17 (br s, 6, $CH_2$), 5.04 (br s, 3, NH).

EXAMPLE 11

Alternative Method for compound (12),

2,4,6-Tris(propargylamino)-1,3,5-triazine.

To a stirred suspension of cyanuric chloride (1.84g, 10 mmol) in dry toluene (50ml) was added dropwise, during 5 min, propargylamine (3.30 g, 60 mmol). When the initial reaction had subsided the mixture was heated under reflux for 20 h, then filtered hot. The solid which separated on cooling (206 mg) was markedly impure (TLC) and was discarded after its removal by filtration. Concentration of the filtrate afforded slightly impure (12) (1.31g) which was recrystallised by boiling with water (100ml) and filtration from undissolved solid to give pure product (773 mg, 32%) as colourless rods identical with material prepared as in Example 9.

EXAMPLE 12

2,4,6-Tris[(hydroxymethyl)(propargyl)amino]-1,3,5-triazine (13).

To a stirred solution of $K_2CO_3$ (414 mg, 3 mmol) in 40% aqueous formaldehyde adjusted to pH 8.9 was added (12) (240 mg, 1 mmol). Complete dissolution occurred in 2 h. After 3 days (13) had separated and was recovered by filtration and washing first with aqueous formaldehyde, then with ice-cold water, then drying in vacuo at 5°C, as a white solid (251 mg, 76%); $\nu_{max}$ (film from $CH_2Cl_2$) 2115 $cm^{-1}$(C≡C str): $\delta_H(CDCl_3)$ 2.25 (t,J=2.5 Hz,3,C≡CH), 4.39 (brs, 6, C≡$CCH_2$), 5.13 (s, 6, $HOCH_2$); m/z (FAB, matrix m-nitrobenzylalcohol-PEG) 331 ($[M+H]^+$).

Analytical data are shown in Table 1 (overleaf).

Table 1: Analytical Data

| COMPOUND | CALCULATED | | | | FOUND | | | |
|---|---|---|---|---|---|---|---|---|
| | C | H | N | F | C | H | N | F |
| 2-fluoro-4,6-bis[(2,2,2-trifluoroethyl) amino]-1,3,5-triazine (2) | 28.68 | 2.06 | 23.89 | 45.37 | 28.80 | 2.17 | 23.52 | 45.34 |
| 2,4,6-tris[(2,2,2-trifluoroethyl)amino]-1,3,5-triazine (3) | 29.07 | 2.44 | 22.60 | 45.89 | 28.90 | 2.42 | 22.55 | 45.97 |
| 2-(hydroxymethyl)(2,2,2-trifluoroethyl) amino-4,6-bis[(2,2,2-trifluoroethyl)amino]-1,3,5-triazine (4) | 29.89 | 2.76 | 20.91 | 42.46 | 29.79 | 2.99 | 20.71 | ----- |
| 2,4,6-tris[(hydroxymethyl)(2,2,2-trifluoroethyl)-amino]-1,3,5-triazine (5) | 31.18 | 3.27 | 18.18 | 36.99 | 31.27 | 3.21 | 17.86 | 36.74 |
| 2-[([hydroxymethoxy]methyl)(2,2,2-trifluoroethyl)amino]-4,6-bis (hydroxymethyl) (2,2,2-trifluoroethyl)amino-1,3,5-triazine (6) | 31.72 | 3.48 | 17.07 | 34.73 | 31.65 | 3.47 | 16.89 | 34.75 |
| 2-(hydroxymethyl)(methyl)amino-4,6-bis[(2,2,2-trifluoroethyl)amino]-1,3,5-triazine (7) | 32.34 | 3.62 | 25.14 | 34.11 | 32.17 | 3.62 | 24.91 | 34.00 |
| 2,4-bis(dimethylamino)-6-(2,2,2-trifluoroethyl)-amino-1,3,5-triazaine (9) | 40.91 | 5.72 | 31.80 | 21.57 | 41.11 | 5.72 | 32.00 | ----- |
| 2,4-bis(dimethylamino)-6-(hydroxymethyl)(2,2,2-trifluoroethyl)amino-1,3,5-triazine (10).$H_2O$ | 38.46 | 6.13 | 26.91 | 18.25 | 38.00 | 5.75 | 26.50 | ----- |
| 2-fluoro-4,6-bis(propargylamino)-1,3,5-triazine (11) | 52.68 | 3.93 | 34.13 | 9.26 | 52.48 | 4.01 | 33.83 | 8.55 |
| 2,4,6-tris(propargylamino)-1,3,5-triazine (12) | 59.99 | 5.03 | 34.98 | ----- | 59.76 | 5.10 | 34.96 | ----- |
| 2,4,6-tris[(hydroxymethyl)(propargyl)amino]-1,3,5-triazine (13) | 54.54 | 5.49 | 25.44 | ----- | 54.41 | 5.50 | 25.27 | ----- |

Stability Studies. The influence of the trifluoromethyl substituent on the stability of the hydroxymethyl function under simulated physiological conditions was determined for compound (10) containing contiguous hydroxymethyl and trifluoroethyl functions.

General Procedure (for 10). To fresh human plasma (9.9 ml) pre-warmed to 37°C was added a solution of the compound to be studied in dimethylsulphoxide (0.1 ml; 10mg/ml) to give a final concentration of 100 μg/ml. At intervals of 0, 5, 10, 15, 20, 30, 40, 50, 60, 75, 90, 105 and 120 min, aliquots (0.5 ml) were withdrawn and added to ice-cold MeOH (1ml). After centrifugation at 4°C the supernatants were placed in cooled vials on a cold plate (3°C) and aliquots (25 μl) analysed by HPLC using an autosampler. The column used was a C6 (Spherisorb) 15cm x 4.6 mm with a precolumn, and was eluted with MeOH:0.05 M aq. $NH_4HCO_3$, 55:45, at a flow rate of 1.5 ml $min^{-1}$. Products were monitored by absorbance at 225 nm. Retention times were: for (10) 11.0 min and for its breakdown product (9) 9.6 min.

The half-life values for the release of product (9) in human plasma at 37°C was 45 min; the corresponding value for N-hydroxymethylpentamethylmelamine was 30 min. Hence, replacement of methyl by trifluoroethyl does give a useful degree of stabilization which should be enhanced by multiple substitution.

Biological Results. The anti-tumour activities of the compounds of the invention against ADJ/PC6 tumour in mice and against human ovarian tumour cell lines were compared with that of trimelamol (1).

Table 2 shows that none of the compounds tested showed in vivo activity, against ADJ/PC6 tumour,comparable with (1). The poor activity relative to (1) appears intrinsic since Table 3, which shows the activity of (5), (6) and (13) against a variety of non-ovarian cell lines shows (1) to be greater than 3-fold more potent against the ADJ/PC6 tumour cell line in vitro than the other compounds.

However, Table 4 shows that the trifluoroethyl analogues (5) and (6) are virtually equipotent with (1) in a variety of ovarian cell lines, as is the propargyl analogue (13). Of particular interest is that compounds (5) and (13) show high activity against cisplatin-resistant cell-lines. The virtually equal potency of compounds (5) and (6) against all cell lines against which both have been tested is consistent with the activity of the latter being wholly accountable for by its spontaneous conversion into (5) in aqueous media such as was demonstrated in the chemical conversion of (6) into (5) in Example 5.

**Table 2: Antitumour Activity Towards the ADJ/PC6 Tumour in Mice of Substituted Melamines.**

| Compound (No.) | LD50 | $ED_{90}$ (mg/kg) | Therapeutic Index $LD_{50}/ED_{90}$ | Highest % inhibition (dose mg/kg) |
|---|---|---|---|---|
| 1 | 140 | 45 | 3.1 | 96 (5 x 100) |
| 3 | 71 | n.a.[a] | - | none[b] |
| 4 | 117 | n.a. | - | 40 (5 x 50) |
| 6 | 280 | n.a. | - | 65 (5 x 12.5) |
| 7 | 140 | n.a. | - | 56 (5 x 25) |
| 13 | 280 | n.a. | - | none |

[a]n.a. - not attained, [b]none - less than 40%.

Table 3: Activities *in vitro* Towards Various Non-Ovarian Cell Lines of Substituted Melamines

| Compound (No.) | PC6 | L-1210 | H69 | GCT | GCT/CIS R |
|---|---|---|---|---|---|
| 1 | 10.1 | 41.7 | 8.7 | 24.3 | 25.9 |
| 5 | 38.2 | 48.6 | ND | ND | ND |
| 6 | 34.4 | ND | ND | 25.8 | 23.7 |
| 13 | 36.8 | 50.7 | ND | 48.9 | 41.8 |

Notes:

| | |
|---|---|
| PC6 | Murine plasmacytoma |
| L-1210 | murine lymphocytic leukaemia |
| H69 | human small cell lung cancer cell line |
| GCT | human testicular germ cell tumour cell line |
| GCT/CIS R | 6-fold platinum resistant variant of above |

Results expressed as μM ($IC_{50}$ values). All experiments were conducted at least in duplicate.

Table 4: Activities <u>in vitro</u> Towards Various Human Ovarian Tumour Cell Lines of Substituted Melamines.

| Compound (No.) | $CH_1$ | $CH_1$ CIS R | 41M | 41M CIS R | HX155 | HX155 CIS R | OvCAR-3 | OvCAR3/CarboR | 2780 | 2780 DDPcis | LK-1 |
|---|---|---|---|---|---|---|---|---|---|---|---|
| | | Δ6 | | Δ5 | | Δ8 | | Δ4 | | Δ12 | |
| 1 | 22.0 | 23.8 | 22.5 | 25.4 | 35.3 | 99.4 | 33.0 | 78.7 | 18.3 | 42.9 | 14.1 |
| 5 | 25.2 | ND | 22.4 | 25.9 | ND | ND | ND | ND | 28.8 | 31.6 | 31.6 |
| 6 | 30.4 | 29.5 | 24.9 | 22.5 | 44.5 | 87.1 | 39.3 | 71.1 | ND | ND | ND |
| 13 | 26.6 | 26.6 | 24.5 | 27.7 | 109.8 | 123.4 | 70.1 | 90.8 | 29.5 | 31.6 | 41.7 |

Notes: LK-1 cell line obtained from a patient treated with Trimelamol
Δ Refers to level of platinum resistance (to cisplatin)

Assay Method: The MTT assay which is in good agreement with cell counting in assessing cytoxic effects of pharmacological agents on cell survival

Results expressed as μM ($IC_{50}$ values)

**Claims**

1. A compound of general formula:

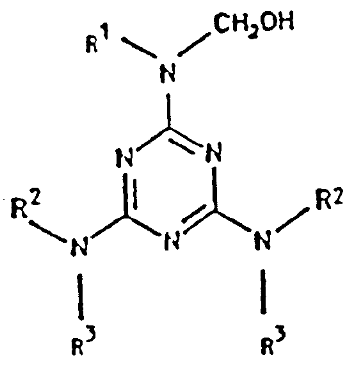

I

wherein $R^3$ is hydrogen, alkyl or hydroxymethyl, and $R^1$ and $R^2$ are hydrogen, alkyl or an electron withdrawing organic group containing at least two carbon atoms, at least one of $R^1$ and $R^2$ being an electron-withdrawing group, and with the proviso that: when $R^3$ is hydroxymethyl, $R^1$ and $R^2$ are electron withdrawing organic groups.

2. A compound according to claim 1 wherein the electron-withdrawing organic group is $CF_3CH_2$- or -$CH_2C{\equiv}CH$.

3. A compound according to claim 1 wherein $R^1$ and each $R^2$ is $CF_3CH_2$- and wherein each $R^3$ is -$CH_2OH$.

4. A compound of general formula:

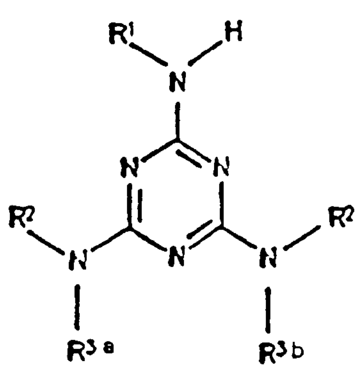

II

wherein $R^{3a}$ and $R^{3b}$ are hydrogen, alkyl or hydroxymethyl, but not both hydroxymethyl, and $R^1$ and $R^2$ are hydrogen, alkyl or an electron withdrawing organic group containing at least two carbon atoms, and at least one of $R^1$ and $R^2$ is an electron-withdrawing organic group.

5. A compound according to claim 4 wherein the electron-withdrawing organic group is $CF_3CH_2$- or -$CH_2C{\equiv}CH$.

6. A compound according to claim 4 wherein $R^1$ and each $R^2$ is $CF_3CH_2$- and wherein $R^{3a}$ and $R^{3b}$ are both H.

7. A compound of the formula:

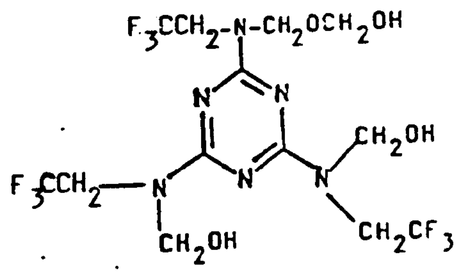

8. A compound according to claim 1, 2 or 3 for use in a method of treatment of the human or animal body by therapy practiced on the human or animal body.

9. A compound according to claim 1, 2 or 3 for use in a method of treatment of cisplatin-resistant ovarian cancer.

10. A pharmaceutical composition comprising an active ingredient which is a compound as defined in claim 1, 2 or 3, together with an inert diluent or carrier.

11. Use of a compound as defined in any one of claims 1, 2 or 3 in the manufacture of a medicament for the treatment of cancer.

12. A process for the preparation of a compound of formula I as defined in any one of claims 1 to 3 which comprises reacting a compound of formula II as defined in any one of claims 4 to 6 with formaldehyde, optionally in the presence of potassium carbonate, and, where the compound of formula II is one in which $R^1$ and each $R^2$ is $CF_3CH_2$- and in which $R^{3a}$ and $R^{3b}$ are both H, treating the resulting reaction product of formula:

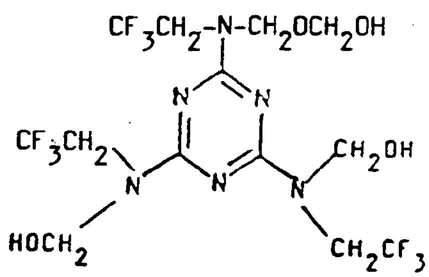

with an aqueous medium.

13. A process for the preparation of a compound as defined in any one of claims 4 to 6 which comprises reacting a cyanuric halide of general formula:
wherein X is fluoro or chloro
with an amine of the formula $R^1NH_2$ or $R^2NH_2$, wherein $R^1$ and $R^2$ are as defined in claim 1, optionally in the presence of caesium fluoride.

14. A process according to claim 13 wherein the cyanuric halide is treated consecutively with two different amines.

European Patent Office

# EUROPEAN SEARCH REPORT

Application Number

EP 92 30 2491

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| | No relevant documents disclosed<br>----- | | C07D251/70<br>A61K31/53 |
| | | | TECHNICAL FIELDS SEARCHED (Int. Cl.5 ) |
| | | | C07D |

The present search report has been drawn up for all claims

| Place of search | Date of completion of the search | Examiner |
|---|---|---|
| THE HAGUE | 26 MAY 1992 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)